# EUROPEAN PATENT SPECIFICATION

(11) **EP 4 209 205 B1**
(45) Date of publication and mention of the grant of the patent: **02.07.2025**
(21) Application number: 22150694.2
(22) Date of filing: 10.01.2022
(51) Int. Cl.: A61H 35/02, A61F 9/00

(54) **EYE RINSING ELEMENT**
AUGENSPÜLELEMENT
ELÉMENT DE RINÇAGE DES YEUX

(43) Date of publication of application: 12.07.2023
(73) Proprietor: Plum Safety ApS, 5610 Assens (DK)
(72) Inventor: BARKHOLT, Bo, Winther, Assens (DK)
(74) Representative: RGTH

(56) References cited:
- DE-U1- 202009 007 205
- US-A- 5 754 990
- US-A1- 2007 119 968
- US-A1- 2016 095 794
- US-S- D 672 870

## Description

The invention relates to an eye rinsing element as well as an eye rinsing device according to the independent claims.

### Prior art

Eye rinsing elements are known from the prior art. These typically have an opening for each eye, from which rinsing fluid can be discharged towards the eyes. However, the stream exiting the eye rinsing elements is typically quite strong, wherein the streams exit the device at a divergence angle such that the distance from the eye to be rinsed to the eye rinsing element is of crucial importance.

DE 20 2009 007 205 U1 as well as US D672,870 S both disclose an eye rinsing device. US 5,754,990 A describes an emergency wash station, while US 2016/0095794 A1 relates to an emergency washing system.

### Description of the invention: objects, solution, advantages

It is an objective of the present invention to improve an eye rinsing element in such a way that a rinsing process of the eyes of a user is simplified, while the process is as comfortable and effective as possible at the same time.

The above objective is solved by providing an eye rinsing element having two rinsing fluid discharge areas, wherein each area comprises multiple, namely 6, openings for discharging rinsing fluid. In particular, each area comprises at least 8, most preferred at least 10, openings for discharging rinsing fluid. In a most preferred embodiment, each area can comprise twelve openings. Especially preferred, each fluid discharge area covers an area of at least 1 cm², more preferred at least 2 cm², most preferred at least 3 cm².

As a result of the above, rinsing fluid is discharged over a wider area than typically known from the prior art and exits the element softer compared to typical eye rinsing elements with just one opening. Especially, the openings discharge fluid in such a way that a beam in each discharge area results which has a diameter of at least 1 mm, further preferred more than 1.1 mm, most preferred more than 1.2 mm. Compared to eye rinsing elements with only one opening per eye, the beam is thus broader and softer which makes the eye rinsing process much more comfortable while at the same time effective and safe for the user.

The openings of each rinsing fluid discharge area are preferably arranged on two concentric circles, namely an inner and an outer circle, wherein the outer circle comprises more openings that the inner circle. Both concentric circles are centered around a central point of the respective fluid discharge area. Advantageously, the outer circle comprises more, especially at least double as many, openings as the inner circle, most preferred exactly double as many openings. Especially preferred, neighboring openings of the inner circle have an angular distance of 90°, wherein neighboring openings of the outer circle have an angular distance of 45°. The openings can further be arranged on at least two concentric circles.

The openings can be formed within the eye rinsing element in a parallel way to each other as well as in a longitudinal direction of the eye rinsing element, so that rinsing fluid is discharged in both discharge areas in the longitudinal direction of the eye rinsing element. In other words, the openings run within the eye rinsing element parallel to each other in a longitudinal direction such that the streams exiting the discharge areas run parallel to each other, independent from the distance to the eye rinsing element. Preferably, each opening has a diameter between 0,1 mm and 5 mm, preferably between 0,5 mm and 1,5 mm, most preferred between 0,8 mm and 1,2 mm. The diameter can also be 1 mm.

According to an invention, in a first longitudinal cross section, the discharge areas form part of a curved surface, which has a radius between 3 mm and 9 mm, preferably between 5 mm and 7 mm, most preferred between 5,5 mm and 6 mm.

Further preferred, the eye rinsing element has a first end area in longitudinal direction comprising the rinsing fluid discharge areas, wherein, in the first end area, the eye rinsing element widens continuously, and preferably constantly, in the first longitudinal cross section. In the respective cross section, the surfaces of the first end area are straight and enclose an angle between 20° and 60°, preferably between 30° and 50°, most preferred around 40°.

In a second longitudinal cross section which is perpendicular to the first longitudinal cross section, the eye rinsing element decreases continuously.

Especially in a second end area in longitudinal direction, the eye rinsing element has at least one, preferably two, constant diameters. In this end area, the eye rinsing element can comprise at least one elevation to indicate a direction in which the eye rinsing element could be attached to a bottle comprising eye rinsing fluid. The elevation can be configured as an arrow. Typically, two elevations can be comprised in the second end area. The second end area is preferably configured as a cap-like portion and can comprise at least one opening, preferably only one opening, for letting rinsing fluid pass from a bottle comprising rinsing fluid to the inside of the eye rinsing element towards the discharge areas. The cap-like portion has preferably the shape of a cap or in other words lit.

The first longitudinal cross section preferably dissects both discharge areas, wherein the second cross section runs between the discharge areas. Especially the eye rinsing element in the second longitudinal cross section decreases consistently. In the respective cross section, the surfaces of the first end area are straight and enclose an angle between 20° and 60°, preferably between 30° and 50°, most preferred around 40°.

The eye rinsing element comprises a discharge channel in its inside guiding rinsing fluid to both discharge areas. Especially the discharge channel connects the opening in the second end area with the discharge areas. In particular, there is no separation separating the discharge channel into two different discharge channels, one for each discharge area inside the eye rinsing element, rather the rinsing fluid follows the geometry as described above and either ends up at one of the discharge areas.

Especially the eye rinsing element is configured as an attachment for an eye rinsing bottle of an eye rinsing device. The eye rinsing bottle preferably comprises eye rinsing fluid and can be squeezed for activating the discharge of eye rinsing fluid and thus an eye rinsing process.

The eye rinsing element can have two recesses which can each serve for hosting a nose of a user of the eye rinsing element. The two recesses are disposed on opposite sides of the element. The recesses are centered on a first plane of symmetry of the eye rinsing element, wherein the first plane of symmetry is disposed between the two discharge areas.

Especially the eye rinsing element has two planes of symmetry, such that each plane of symmetry cuts the eye rinsing elements into two mirrored halves, preferably except for the elevations in the second end area. The two planes of symmetry are perpendicular to each other, and both run in the longitudinal direction of the eye rinsing element. While the first plane of symmetry is disposed between the two discharge areas and comprises the first cross section, the second plane of symmetry runs through both discharge areas and comprises the second longitudinal cross section. In other words, the eye rinsing element has an inwardly curved configuration forming the recesses in a central part of both sides of the eye rinsing element, so that a user has two different possibilities to put the eye rinsing element to the middle of his nose.

In a further aspect, the invention relates to an eye rinsing device comprising an eye rinsing bottle and an eye rinsing element as described above.

### Brief description of the drawings

The invention will be described below with reference to the following figures which show in schematic representation:
- Figure 1:: a perspective view of an eye rinsing element;
- Figure 2:: a top view of a first end area of the eye rinsing element according to figure 1;
- Figure 3:: a cross-sectional view along the section A-A of figure 2;
- Figure 4:: a side view of the eye rinsing element according to the proceeding figures;
- Figure 5:: a cross-sectional view along the lines B-B of figure 2; and
- Figure 6:: another side view of the eye rinsing element.

### Preferred embodiments of the invention

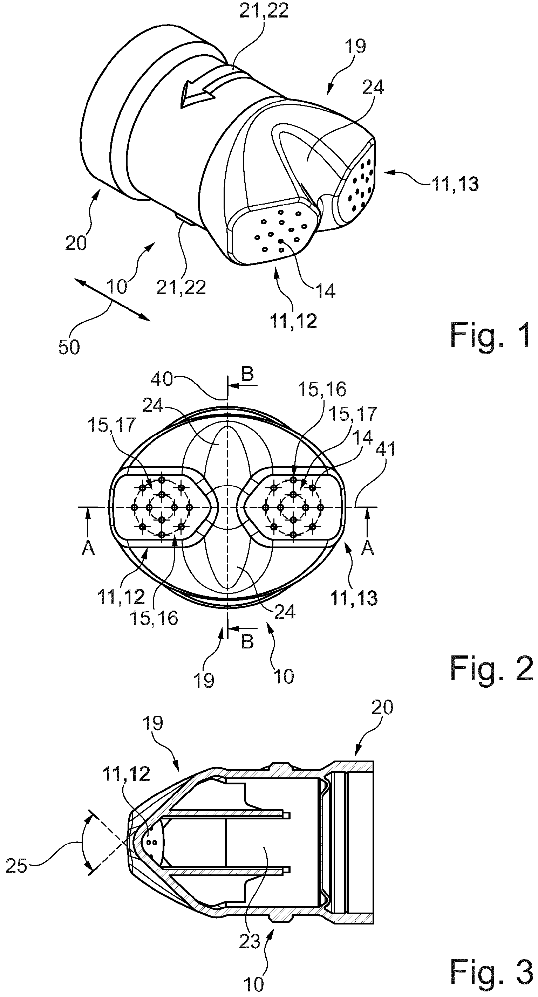

Figure 1 shows a perspective view of an eye rinsing element 10 according to the present invention. The eye rinsing element 10 has two rinsing fluid discharge areas 11, namely a first discharge area 12 as well as a second discharge area 13. Each discharge area 11 comprises multiple openings 14.

In a longitudinal direction 15 of the eye rinsing element 10, the eye rinsing element 10 has two end areas, namely a first end area 19, in which the discharge areas 11 are disposed, as well as a second end area 20 which is configured as a cap-like portion for being attached to an eye rinsing bottle comprising eye rinsing fluid. In the second end area 20, two elevations 21 in the form of an arrow 22 are disposed to indicate a direction to screw the eye rinsing element onto an eye rinsing bottle.

In figure 1 a recess 24 in the first end area 19 for hosting a nose of a user can clearly be seen, which extends towards the end of the eye rinsing element 10 in the first end area 19.

Figure 2 shows a top view on the first end area 19 of the eye rinsing element 10 according to figure 1. Clearly the two discharge areas 11 can be seen comprising the openings 14. The openings 14 are disposed on two concentric circles 15, namely an outer circle 16 and an inner circle 17. While the inner circle 17 has four openings, the outer circle 16 has double as much, namely eight, openings 14. The eye rinsing element 10 has two planes of symmetry, namely a first plane 40 of symmetry and a second plane 41 of symmetry. Both planes of symmetry run in the longitudinal direction 15. Except for the elevation 21 in the second end area 20, the planes of symmetry divide the eye rinsing element 10 into two mirrored halves. The recesses 24 are centered on the first plane of symmetry 40.

The first plane of symmetry 40 runs between the two discharge areas 11, while the second plane of symmetry 41 runs through both of them. The two planes of symmetry are perpendicular to each other. Thus, in the top view seen in figure 2 the recesses 24 are formed in a middle portion towards the first plane 40 of symmetry allowing the nose of a user to be hosted into a recess so that the eye rinsing element 10 can be used in two different orientations.

Figure 3 shows a longitudinal cross-section along the lines A-A of figure 2. This longitudinal cross-section is the second longitudinal cross-section. The eye rinsing element 10 decreases in its first end area 19 starting from the second end area 20 to a free end of the eye rinsing element. The eye rinsing element 10 decreases continuously, while the surfaces enclose an angle 25 of roughly 40°. In the inside of the eye rinsing element 10, a discharge channel 23 is configured allowing rinsing fluid to either flow towards the first discharge area 12 or the second discharge area 13.

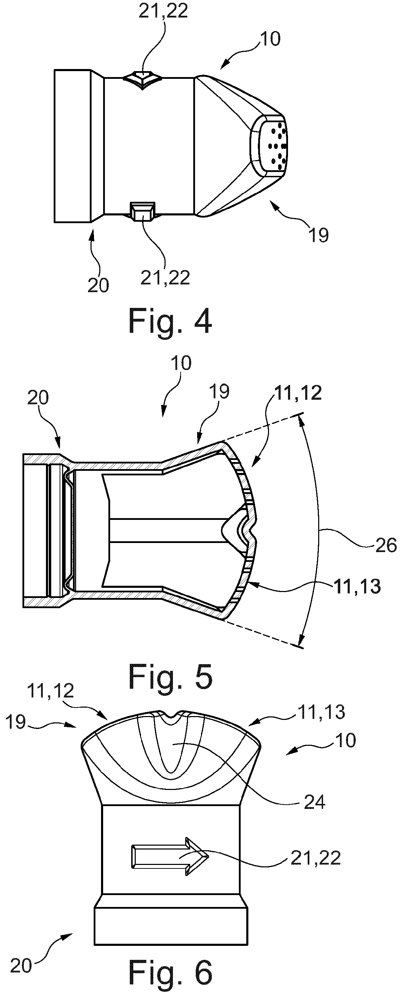

Figure 4 shows a side view of the eye rinsing element 10 in which again the decrease of the eye rinsing element starting from the second end area 20 towards the discharge areas 11 is seen. In the second end area 20, the eye rinsing element has two constant diameters, one being larger at the free end of the eye rinsing element 10 in the second end area, wherein the elevation is disposed in the section with the smaller constant diameter.

In figure 5 a cross-section along the lines A-A of figure 2 is shown. This is the first longitudinal cross-section in which the eye rinsing element 10 within its first end area 19 widens continuously such that the surfaces of the eye rinsing element in the cross-section have an angle 26 to each other.

Figure 6 shows another side view of the eye rinsing element 10 with its first end area 19 and its second end area 20. Again, the discharge areas 11, the first discharge area 12 as well as the second discharge area 13, can be seen from the side. In between extending towards the middle there is the recess 24.

### List of reference signs

- 10: eye rinsing element
- 11: discharge area
- 12: first discharge area
- 13: second discharge area
- 14: opening
- 15: concentric circle
- 16: outer circle
- 17: inner circle
- 18: curved surface
- 19: first end area
- 20: second end area
- 21: elevation
- 22: arrow
- 23: discharge channel
- 24: recess
- 25: first angle
- 26: second angle

- 40: first plane of symmetry
- 41: second plane of symmetry

- 50: longitudinal direction

## Claims

1. An eye rinsing element (10),
wherein the eye rinsing element (10) comprises two rinsing fluid discharge areas (11),
**characterized in that**
the discharge areas (11) form part of a curved surface which in a first longitudinal cross-section (A-A) has a radius between 3 mm and 9 mm,
and wherein each discharge area (11) comprises at least 6 openings (14) for discharging rinsing fluid.

2. The eye rinsing element (10) according to claim 1,
**characterized in that**
the discharge areas (11) each comprise at least 8, most preferred at least 10, openings (14).

3. The eye rinsing element (10) according to any one of claims 1 or 2,
**characterized in that**
the openings (14) are arranged on two concentric circles.

4. The eye rinsing element (10) according to claim 3,
**characterized in that**
an outer circle (16) comprises more openings (14) than an inner circle (17).

5. The eye rinsing element (10) according to claim 4,
**characterized in that**
the outer circle (16) comprises at least double as many openings (14) as the inner circle (17).

6. The eye rinsing element (10) according to any one of claims 4 or 5,
**characterized in that**
neighbouring openings (14) of the inner circle (17) have an angular distance of 90°,
wherein neighbouring openings (14) of the outer circle (16) have an angular distance of 45°.

7. The eye rinsing element (10) according to any one of the preceding claims,
**characterized in that**
the openings (14) are formed within the eye rinsing element (10) parallel to each other and in a longitudinal direction (50) of the eye rinsing element (10),
so that rinsing fluid is discharged in both discharge areas (11) in the longitudinal direction (50) of the eye rinsing element (10).

8. The eye rinsing element (10) according to any one of the preceding claims,
**characterized in that**
the openings (14) have a diameter between 0,1 mm and 5 mm.

9. The eye rinsing element (10) according to any one of the preceding claims,
**characterized in that**
the eye rinsing element (10) has a first end area (19) in longitudinal direction (50) comprising the rinsing fluid discharge areas (11),
wherein in the first end area (19) the eye rinsing element (10) widens continuously in a first longitudinal cross-section.

10. The eye rinsing element (10) according to claim 9,
**characterized in that**
in the first end area (19), the eye rinsing element (10) decreases continuously in a second longitudinal cross-section,
wherein the second longitudinal section is perpendicular to the first longitudinal cross-section.

11. The eye rinsing element (10) according to any one of the preceding claims,
**characterized in that**
the eye rinsing element (10) comprises a discharge channel (23) in its inside guiding rinsing fluid to both discharge areas (11).

12. The eye rinsing element (10) according to any one of the preceding
claims,
**characterized in that**
the eye rinsing element (10) has two recesses for a nose of a user of the eye rinsing element (10),
said two recesses (24) being disposed on opposite sides of the eye rinsing element (10),
wherein the recesses (24) are centred on a first plane (40) of symmetry of the eye rinsing element (10),
the first plane (40) of symmetry being disposed between the two discharge areas (11).

13. The eye rinsing element (10) according to any one of the preceding claims,
**characterized in that**
the eye rinsing element (10) is configured as an attachment for an eye rinsing bottle of an eye rinsing device.

14. Eye rinsing device comprising an eye rinsing bottle and an eye rinsing element (10) according to claims 1 to 13.

## Patentansprüche

1. Augenspülelement (10),
wobei das Augenspülelement (10) zwei Spülflüssigkeitsauslassbereiche (11) umfasst,
**dadurch gekennzeichnet, dass**
die Auslassbereiche (11) einen Teil einer gekrümmten Fläche ausbilden, die in einem ersten Längsschnitt (A-A) einen Radius zwischen 3 mm und 9 mm aufweist,
und wobei jeder Auslassbereich (11) mindestens 6 Öffnungen (14) zum Auslassen von Spülflüssigkeit umfasst.

2. Augenspülelement (10) gemäß Anspruch 1,
**dadurch gekennzeichnet, dass**
die Auslassbereiche (11) jeweils mindestens 8, besonders bevorzugt mindestens 10, Öffnungen (14) umfassen.

3. Augenspülelement (10) gemäß einem der Ansprüche 1 oder 2,
**dadurch gekennzeichnet, dass**
die Öffnungen (14) auf zwei konzentrischen Kreisen angeordnet sind.

4. Augenspülelement (10) gemäß Anspruch 3,
**dadurch gekennzeichnet, dass**
ein Außenkreis (16) mehr Öffnungen (14) als ein Innenkreis (17) umfasst.

5. Augenspülelement (10) gemäß Anspruch 4,
**dadurch gekennzeichnet, dass**
der Außenkreis (16) mindestens doppelt so viele Öffnungen (14) umfasst wie der Innenkreis (17).

6. Augenspülelement (10) gemäß einem der Ansprüche 4 oder 5,
**dadurch gekennzeichnet, dass**
benachbarte Öffnungen (14) des Innenkreises (17) einen Winkelabstand von 90° aufweisen,
wobei benachbarte Öffnungen (14) des Außenkreises (16) einen Winkelabstand von 45° aufweisen.

7. Augenspülelement (10) gemäß einem der vorhergehenden Ansprüche,
**dadurch gekennzeichnet, dass**
die Öffnungen (14) innerhalb des Augenspülelements (10) parallel zueinander und in einer Längsrichtung (50) des Augenspülelements (10) ausgebildet sind, so dass die Spülflüssigkeit in beiden Auslassbereichen (11) in Längsrichtung (50) des Augenspülelements (10) ausgelassen wird.

8. Augenspülelement (10) gemäß einem der vorhergehenden Ansprüche,
**dadurch gekennzeichnet, dass**
die Öffnungen (14) einen Durchmesser zwischen 0,1 mm und 5 mm aufweisen.

9. Augenspülelement (10) gemäß einem der vorhergehenden Ansprüche,
**dadurch gekennzeichnet, dass**
das Augenspülelement (10) einen ersten Endbereich (19) in Längsrichtung (50) aufweist, der die Spülflüssigkeitsauslassbereiche (11) umfasst,
wobei sich das Augenspülelement (10) im ersten Endbereich (19) in einem ersten Längsquerschnitt kontinuierlich erweitert.

10. Augenspülelement (10) gemäß Anspruch 9,
**dadurch gekennzeichnet, dass**
sich das Augenspülelement (10) im ersten Endbereich (19) in einem zweiten Längsschnitt kontinuierlich verringert,
wobei der zweite Längsschnitt senkrecht zum ersten Längsquerschnitt verläuft.

11. Augenspülelement (10) gemäß einem der vorhergehenden Ansprüche,
**dadurch gekennzeichnet, dass**
das Augenspülelement (10) in seinem Inneren einen Auslasskanal (23) umfasst, der Spülflüssigkeit zu beiden Auslassbereichen (11) leitet.

12. Augenspülelement (10) gemäß einem der vorhergehenden Ansprüche,
**dadurch gekennzeichnet, dass**
das Augenspülelement (10) zwei Aussparungen für eine Nase eines Benutzers des Augenspülelements (10) aufweist,
wobei die beiden Aussparungen (24) auf gegenüberliegenden Seiten des Augenspülelements (10) angeordnet sind,
wobei die Aussparungen (24) auf einer ersten Symmetrieebene (40) des Augenspülelements (10) zentriert sind,
wobei die erste Symmetrieebene (40) zwischen den beiden Auslassbereichen (11) angeordnet ist.

13. Augenspülelement (10) gemäß einem der vorhergehenden Ansprüche,
**dadurch gekennzeichnet, dass**
das Augenspülelement (10) als Aufsatz für eine Augenspülflasche einer Augenspülvorrichtung konfiguriert ist.

14. Augenspülvorrichtung umfassend eine Augenspülflasche und ein Augenspülelement (10) gemäß einem der Ansprüche 1 bis 13.

## Revendications

1. Un élément de rinçage des yeux (10),
dans lequel l'élément de rinçage des yeux (10) comprend deux zones d'évacuation du liquide de rinçage (11),
**caractérisé par le fait que**
les zones d'évacuation (11) font partie d'une surface courbe qui, dans une première section transversale longitudinale (A-A), a un rayon compris entre 3 mm et 9 mm,
et dans lequel chaque zone d'évacuation (11) comprend au moins 6 ouvertures (14) pour l'évacuation du liquide de rinçage.

2. L'élément de rinçage des yeux (10) selon la revendication 1,
**caractérisé par le fait que**
les zones d'évacuation (11) comprennent chacune au moins 8, de préférence au moins 10, ouvertures (14).

3. L'élément de rinçage des yeux (10) selon l'une quelconque des revendications 1 ou 2,
**caractérisé par le fait que**
les ouvertures (14) sont disposées sur deux cercles concentriques.

4. L'élément de rinçage des yeux (10) selon la revendication 3,
**caractérisé par le fait que**
un cercle extérieur (16) comporte plus d'ouvertures (14) qu'un cercle intérieur (17).

5. L'élément de rinçage des yeux (10) selon la revendication 4,
**caractérisé par le fait que**
le cercle extérieur (16) comporte au moins deux fois plus d'ouvertures (14) que le cercle intérieur (17).

6. L'élément de rinçage des yeux (10) selon l'une quelconque des revendications 4 ou 5,
**caractérisé par le fait que**
des ouvertures voisines (14) du cercle intérieur (17) ont une distance angulaire de 90°,
dans lequel des ouvertures voisines (14) du cercle extérieur (16) ont une distance angulaire de 45°.

7. L'élément de rinçage des yeux (10) selon l'une quelconque des revendications précédentes,
**caractérisé par le fait que**
les ouvertures (14) sont formées à l'intérieur de l'élément de rinçage des yeux (10) parallèlement les unes aux autres et dans une direction longitudinale (50) de l'élément de rinçage des yeux (10),
de manière à ce que le liquide de rinçage soit évacué dans les deux zones d'évacuation (11) dans la direction longitudinale (50) de l'élément de rinçage des yeux (10).

8. L'élément de rinçage des yeux (10) selon l'une quelconque des revendications précédentes,
**caractérisé par le fait que**
les ouvertures (14) ont un diamètre compris entre 0,1 mm et 5 mm.

9. L'élément de rinçage des yeux (10) selon l'une quelconque des revendications précédentes,
**caractérisé par le fait que**
l'élément de rinçage des yeux (10) a une première zone d'extrémité (19) dans la direction longitudinale (50) comprenant les zones d'évacuation du liquide de rinçage (11),
dans lequel, dans la première zone d'extrémité (19), l'élément de rinçage des yeux (10) s'élargit continuellement dans une première section transversale longitudinale.

10. L'élément de rinçage des yeux (10) selon la revendication 9,
**caractérisé par le fait que**
dans la première zone d'extrémité (19), l'élément de rinçage des yeux (10) diminue continuellement dans une deuxième section transversale longitudinale,
la seconde section longitudinale est perpendiculaire à la première section transversale longitudinale.

11. L'élément de rinçage des yeux (10) selon l'une quelconque des revendications précédentes,
**caractérisé par le fait que**
l'élément de rinçage des yeux (10) comprend un canal d'évacuation (23) à l'intérieur duquel du liquide de rinçage est acheminé vers les deux zones d'évacuation (11).

12. L'élément de rinçage des yeux (10) selon l'une quelconque des revendications précédentes,
**caractérisé par le fait que**
l'élément de rinçage des yeux (10) comporte deux évidements pour un nez d'un utilisateur de l'élément de rinçage des yeux (10),
ces deux évidements (24) sont disposés sur des côtés opposés de l'élément de rinçage des yeux (10),
dans lequel les évidements (24) sont centrés sur un premier plan (40) de symétrie de l'élément de rinçage des yeux (10),
le premier plan (40) de symétrie est disposé entre les deux zones d'évacuation (11).

13. L'élément de rinçage des yeux (10) selon l'une quelconque des revendications précédentes,
**caractérisé par le fait que**
l'élément de rinçage des yeux (10) est configuré comme un accessoire pour un flacon de rinçage des yeux d'un dispositif de rinçage des yeux.

14. Dispositif de rinçage des yeux comprenant un flacon de rinçage des yeux et un élément de rinçage des yeux (10) selon les revendications 1 à 13.
